(19) Europäisches Patentamt
European Patent Office
Office européen des brevets

(11) **EP 4 609 889 A1**

(12) **EUROPEAN PATENT APPLICATION**
published in accordance with Art. 153(4) EPC

(43) Date of publication:
**03.09.2025 Bulletin 2025/36**

(21) Application number: **22963376.3**

(22) Date of filing: **24.10.2022**

(51) International Patent Classification (IPC):
**A61M 1/16** (2006.01)

(52) Cooperative Patent Classification (CPC):
**A61M 1/16**

(86) International application number:
**PCT/JP2022/039439**

(87) International publication number:
**WO 2024/089724 (02.05.2024 Gazette 2024/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Shibuya Corporation
Kanazawa-shi, Ishikawa 920-8681 (JP)**

(72) Inventors:
• **MATSUZAKI Kosho
Kanazawa-shi, Ishikawa 920-8681 (JP)**
• **FUJII Masayuki
Kanazawa-shi, Ishikawa 920-8681 (JP)**

(74) Representative: **Mewburn Ellis LLP
Aurora Building
Counterslip
Bristol BS1 6BX (GB)**

(54) **HEMODIALYSIS DEVICE**

(57) When a pressure change suddenly occurs, a replacement fluid is quickly stabilized.

Provided is a hemodialysis device 1 including a replacement fluid passage 5 between a dialysate circuit 4 and a blood circuit 3, the dialysate circuit 4 and the replacement fluid passage 5 being respectively provided with first and second flow rate restriction device MV1 and MV2.

A connection portion pressure sensor PG2 for measuring a pressure of a connection portion between a dialysate supply passage 14 and the replacement fluid passage 5, and a vein-side pressure sensor PV (blood circuit pressure measurement device) for measuring a pressure of the blood circuit 3 are included.

The control device 6 adjusts an opening degree of the second flow rate restriction device MV2 a plurality of times on the basis of the measured pressure of the connection portion pressure sensor PG2, and the measured pressure of the vein-side pressure sensor PV, for one round, defined as being from when a supply compartment of a chamber provided to the dialysate circuit 4 is filled with a fresh dialysate until all of the fresh dialysate in the supply compartment has been discharged (shown by white rhombuses in the drawing).

Fig. 1

EP 4 609 889 A1

## Description

[Technical Field]

The present invention relates to a hemodialysis device, and more particularly relates to a hemodialysis device that performs fluid replacement via a replacement fluid passage provided between a dialysate circuit and a blood circuit.

[Background Art]

Conventionally, it has been known that fluid replacement is performed via a replacement fluid passage provided between a dialysate circuit and a blood circuit during hemodialysis using a hemodialysis device, and as such a hemodialysis device that performs fluid replacement, there has been known a hemodialysis device in which first and second flow rate restriction device are provided respectively in a dialysate circuit and a replacement fluid passage (Patent Literature 1).

In Patent Literature 1 described above, control device controls the above-described first and second flow rate restriction device so that the pressure of a connection portion of a dialysate supply passage and the replacement fluid passage becomes higher than the pressure of the blood circuit, and supplies the dialysate of the dialysate circuit to the blood circuit.

[Prior Art Documents]

[Patent Literature]

[Patent Literature 1]
Japanese Laid-Open Patent Application No. 2021-62067

[Summary of Invention]

[Problems to be Solved by the Invention]

However, Patent Literature 1 described above does not take into account the occurrence of a sudden pressure change in the blood circuit, for example, occurrence of a so-called kink, in which a tube composing the blood circuit is bent due to a change in the posture or the like of a patient.

For example, when the above-described kink occurs at an upstream side of the connection portion with the above-described replacement fluid passage in the blood circuit, pressure at a downstream side of the connection portion becomes relatively negative pressure, so that more dialysate than a target replacement fluid flow rate that is set in advance is likely to flow into the blood circuit, and in this case, it is necessary to quickly stabilize the replacement fluid.

In view of the problem as above, the present invention provides a hemodialysis device that can quickly stabilize a replacement fluid even when a pressure change in a blood circuit suddenly occurs.

[Means for Solving the Problems]

A hemodialysis device according to the invention of claim 1 is a hemodialysis device including a dialyzer that performs hemodialysis, a chamber that is provided in a dialysate circuit and stores a dialysate, a dialysate supply passage that is connected to a supply compartment of the chamber to supply a fresh dialysate to the dialyzer, a dialysate recovery passage that is connected to a recovery compartment of the chamber to recover a used dialysate that flows through the dialyzer, a blood circuit that causes blood to flow through the dialyzer, a replacement fluid passage provided between the dialysate supply passage and the blood circuit, first flow rate restriction device provided at a downstream side of a connection portion with the replacement fluid passage in the dialysate supply passage, second flow rate restriction device provided in the replacement fluid passage, and control device that controls the first flow rate restriction device and the second flow rate restriction device,

the control device adjusting a flow rate of a replacement fluid that is supplied to the blood circuit via the replacement fluid passage by controlling the first flow rate restriction device and the second flow rate restriction device, characterized by including
connection portion pressure measurement device that measures a pressure of the connection portion of the dialysate supply passage and the replacement fluid passage, and blood circuit pressure measurement device that measures a pressure of the blood circuit, and characterized in that

during one round from a time when the supply compartment of the chamber is in a state of being filled with the fresh dialysate until all of the fresh dialysate in the supply compartment is discharged, the control device

performs feedback control that adjusts the second flow rate restriction device a plurality of times based on a replacement fluid flow rate set as a target, a measured pressure of the connection portion pressure measurement device, and a measured pressure of the blood circuit pressure measurement device.

[Advantageous Effects of Invention]

According to the invention of claim 1, even when a sudden pressure change occurs, the replacement fluid flow rate is quickly adjusted by executing the feedback control on the above-described second flow rate restriction device a plurality of times during one round, and therefore stable fluid replacement is possible.

[Brief Description of Drawings]

[Figure 1] Figure 1 is a circuit diagram showing an embodiment of the present invention.
[Figure 2] Figure 2 is a diagram for explaining an operation when only second feedback control in the present embodiment is performedr
[Figure 3] Figure 3 is a diagram for explaining an operation when first and second feedback control in the present embodiment are performed.

[Mode for Carrying out the Invention]

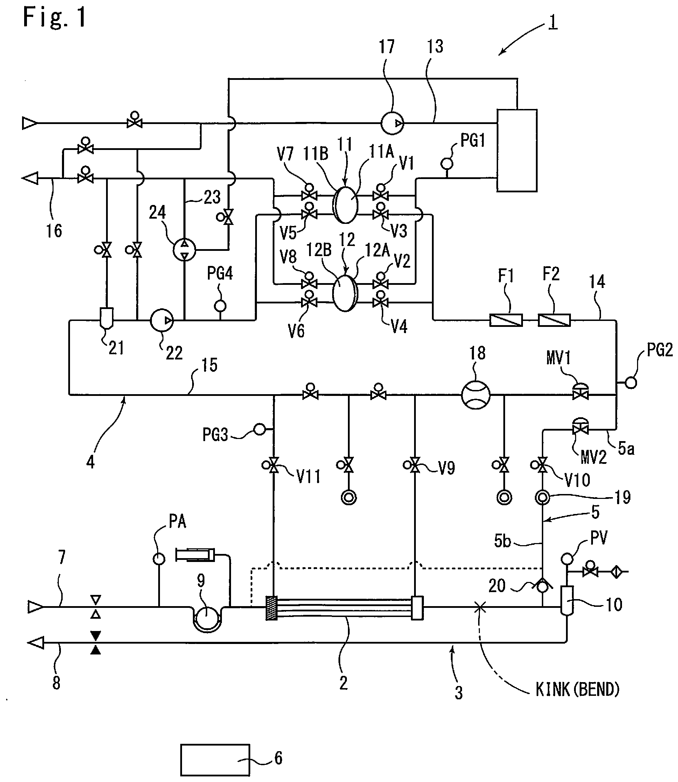

Explaining the present invention concerning an embodiment shown in the drawings hereinafter, in Figure 1, a hemodialysis device 1 includes a blood circuit 3 which is connected to a dialyzer 2 and through which blood flows, a dialysate circuit 4 which is connected to the dialyzer 2 and through which a dialysate passes, and a replacement fluid passage 5 that allows the blood circuit 3 and the dialysate circuit 4 to communicate with each other.

The above-described hemodialysis device 1 is controlled by control device 6, and as the control device 6, it is possible to use a personal computer, a microcomputer, PLC, or the like, which contains a microprocessor and controls each device composing the above-described hemodialysis device 1 by a user-changeable program.

The above-described blood circuit 3 comprises an artery-side passage 7 that is connected to a blood vessel of a patient to supply blood to the above-described dialyzer 2, and a vein-side passage 8 that returns blood to the patient from the dialyzer 2.

In the above-described artery-side passage 7, a distal end thereof is punctured into an artery of the patient, and a proximal end is connected to an inlet of the dialyzer 2. Further, in the artery-side passage 7, an artery-side pressure sensor PA, and a blood pump 9 that delivers blood are provided.

In the above-described vein-side passage 8, a proximal end is connected to an outlet of the above-described dialyzer 2, and a distal end is punctured into a vein of the patient. Further, in the vein-side passage 8, a drip chamber 10 is provided, and a vein-side pressure sensor PV as blood circuit pressure measurement device is provided at the drip chamber 10.

In the above-described dialysate circuit 4, a first chamber 11 and a second chamber 12 having a same shape and storing a dialysate are provided. In these first and second chambers 11 and 12, insides thereof are respectively divided into two compartments by flexible diaphragms, one compartments are supply compartments 11A and 12A that store a fresh dialysate, and the other compartments are recovery compartments 11B and 12B that recover a used dialysate.

To the above-described supply compartments 11A and 12A, a supply fluid passage 13 supplied with the fresh dialysate, and a dialysate supply passage 14 for supplying the fresh dialysate to the dialyzer 2 are respectively branched and connected. Fluid supply valves V1 and V2 are provided in branched passages of the supply fluid passage 13, and supply valves V3 and V4 are provided in branched passages of the dialysate supply passage 14.

To the above-described recovery compartments 11B and 12B, a dialysate recovery passage 15 for recovering the used dialysate that flows through the dialyzer 2, and a drainage passage 16 that discharges the used dialysate are connected. Recovery valves V5 and V6 are provided in branched passages of the dialysate recovery passage 15, and drainage valves V7 and V8 are provided in branched passages of the drainage passage 16.

In the above-described supply fluid passage 13, a fresh dialysate supply device not shown that supplies the fresh dialysate is provided at an upstream side thereof, and A fluid supply pump 17 and a fluid supply pressure sensor PG1 are provided between the first and second chambers 11 and 12.

In the above-described dialysate supply passage 14, a first end toxin cut filter F1 and a second end toxin cut filter F2 that clean the dialysate, and a connection portion pressure sensor PG2 as connection portion pressure measurement device according to the present invention are provided, and the above-described replacement fluid passage 5 is connected to a downstream side of the connection portion pressure sensor PG2.

At a downstream side of the connection portion with the above-described replacement fluid passage 5 in the dialysate supply passage 14, first flow rate restriction device MV1 controlled by the control device 6, a flowmeter 18, and an on-off valve V9 are provided.

The replacement fluid passage 5 of the present embodiment is configured by connecting pipes branched from the dialysate circuit 4 and the blood circuit 3 to each other.

Specifically, a replacement fluid passage 5a on a dialysate circuit 4 side is provided by being branched from between the connection portion pressure sensor PG2 and the first flow rate restriction device MV1 in the above-described dialysate supply passage 14 as described above.

In the replacement fluid passage 5a on the dialysate circuit 4 side, second flow rate restriction device MV2 controlled by the control device 6, an on-off valve V10, and a connection port 19 for connecting to the replacement fluid passage 5a on an above-described blood circuit 3 side are provided.

On the other hand, a replacement fluid passage 5b on the blood circuit 3 side is provided by being branched from between the above-described dialyzer 2 and the drip chamber 10 in the above-described vein-side passage 8, and a distal end portion is connected to the above-described connection port 19.

In the replacement fluid passage 5b on the blood circuit 3 side, a check valve 20 for preventing backflow of blood from the blood circuit 3 is provided at a position adjacent to a branched portion.

Here, a pipe configuration in which the replacement fluid passage 5b is branched from the vein-side passage 8 is referred to as post-connection, a pipe configuration in which the replacement fluid passage 5b is branched from the artery-side passage 7 as shown by a broken line is referred to as pre-connection, and the feedback control according to the present invention is compatible with either of the connection methods.

In the present embodiment, conventional well-known diaphragm valves that can be controlled by the control device 6 are used for the above-described first and second flow rate restriction device MV1 and MV2.

The flow rate restriction device using the diaphragm valve increases or decreases a flow path area by increasing or decreasing a lift amount of the diaphragm valve with respect to a valve seat to adjust the flow rate of the circulating dialysate, and the lift amount can be adjusted by a voltage applied by the control device 6.

In the above-described dialysate recovery passage 15, in order from the upstream side corresponding to the dialyzer 2 side, an on-off valve V11, a first recovery side pressure sensor PG3, a deaeration tank 21 that removes bubbles in the dialysate, a fluid delivery pump 22 that feeds the used dialysate to the respective recovery compartments 11B and 12B of the first and second chambers 11 and 12, and a second recovery side pressure sensor PG4 are provided.

A fluid removal passage 23 is connected between the above-described fluid delivery pump 22 and the second recovery side pressure sensor PG4, and a required amount of used dialysate is discharged into the drainage passage 16 via a fluid removal pump 24 provided in the fluid removal passage 23, and fluid removal is performed during dialysis treatment.

An operation of the hemodialysis device 1 having the above-described configuration will be described. Note that in the following explanation, a fluid replacement operation will be mainly described, and detailed explanation of the other operations will be omitted since the other operations are conventionally known.

First of all, before starting hemodialysis by the hemodialysis device 1, the artery-side passage 7 and the vein-side passage 8 of the blood circuit 3 are connected to a patient respectively, and various settings are performed in advance.

In the present embodiment, the doctor sets the dialysate flow rate and the replacement fluid flow rate according to physical conditions or the like of the patient, and here, as an example, a target dialysate flow rate Dtf of a fresh dialysate to be circulated from the above-described dialysate supply passage 14 to the dialyzer 2 is set to 700 ml/min, while a target replacement fluid flow rate Stf for performing fluid replacement for the patient via the replacement fluid passage 5 is set to 100 ml/min.

When setting as above is finished, and a command for starting hemodialysis is inputted to the control device 6, in the dialysate circuit 4, the fresh dialysate is supplied from the above-described supply fluid passage 13 to the supply compartment 11A of the first chamber 11, and the volume of the supply compartment 11A is enlarged by deformation of the diaphragm. As a result, a volume of the recovery compartment 11B is reduced, and the used dialysate stored in the recovery compartment 11B is discharged into the drainage passage 16.

At the same time, the used dialysate that passes through the dialyzer 2 flows through the dialysate recovery passage 15 and is recovered into the recovery compartment 12B of the second chamber 12. With this, the fresh dialysate of the supply compartment 12A is supplied to the dialyzer 2 via the dialysate supply passage 14 by deformation of the diaphragm.

Thereafter, the control device 6 switches open/closed states of the above-described fluid supply valves V1 and V2 and drainage valves V7 and V8, and supply valves V3 and V4 and recovery valves V5 and V6 to thereby alternately supply the fresh dialysate to the dialyzer 2 from the first and second chambers 11 and 12.

Here, for example, in the first chamber 11, a period from a time when the volume of the supply compartment 11A is in the state of being maximized to a time when all of the fresh dialysate in the supply compartment 11A is discharged and the volume becomes zero is referred to as one round.

A time period required for the above-described one round can be detected by using the second recovery side pressure sensor PG4 provided in the above-described dialysate recovery passage 15.

Explaining specifically, when the used dialysate flows into the recovery compartment 12B of the second chamber 12, and thereafter the volume of the supply compartment 12A becomes zero due to the deformation of the diaphragm, a pressure wave (water hammer) is generated, and the shock wave is detected by the above-described second recovery side pressure sensor PG4.

Then, the control device 6 recognizes that delivery of the dialysate from the second chamber 12 is completed, switches the recovery valves V5 and V6 and the drainage valves V7 and V8 of the above-described first and second chambers 11 and 12, and sets the time point when the fluid delivery is started from the supply compartment 11A of the first chamber 1 as a fluid delivery start time point.

Thereafter, when the control device 6 detects that the fluid delivery from the supply compartment 11A of the first chamber 11 is completed by the above-described second recovery side pressure sensor PG4, the control device 6 recognizes the time point thereof as a fluid delivery completion time point.

As described above, the control device 6 recognizes the time period from the fluid delivery start time point of the supply compartment 11A of the above-described first chamber 11 to the fluid delivery start time point of the supply compartment 12A of the above-described second chamber 12 as one round.

In other words, the one round means the period that starts from the time point when the fluid delivery of the supply compartment of the one chamber is started until the time point when fluid delivery of the supply compartment of the other chamber starts after inflow of the used dialysate to the recovery compartment of the one chamber is completed.

Next, when fluid replacement for the patient is performed during the dialysis treatment in the hemodialysis device 1, the control device 6 opens the on-off valve V10 that is provided in the replacement fluid passage 5. Then, a part of the dialysate that is supplied to the dialyzer 2 from the dialysate supply passage 14 is supplied to the blood circuit 3 via the replacement fluid passage 5, and fluid replacement for the patient is performed.

At this time, the control device 6 controls the first flow rate restriction device MV1 provided in the dialysate supply passage 14, and the second flow rate restriction device MV2 provided in the replacement fluid passage 5 to cause the dialysate at the above-described target replacement fluid flow rate Stf to flow through the replacement fluid passage 5.

Then, the dialysate that flows into the blood circuit 3 from the replacement fluid passage 5 is supplied to the patient as the replacement fluid. At this time, backflow from the blood circuit 3 to the replacement fluid passage 5 is prevented by the check valve 20 provided in the above-described replacement fluid passage 5.

Here, when the dialysate is supplied from the dialysate circuit 4 to the blood circuit 3 via the above-described replacement fluid passage **5,** the flow rate of the replacement fluid that flows into the blood circuit 3 from the replacement fluid passage 5 may change due to the change in the pressure of the blood flowing through the blood circuit 3.

For example, when the pressure of the blood that flows through the blood circuit 3 is high, inflow of the dialysate from the replacement fluid passage 5 is hindered, and therefore, less dialysate than the target replacement fluid flow rate Stf is administered. On the other hand, when the pressure of the blood that flows through the blood circuit 3 is low, the dialysate excessively flows in from the replacement fluid passage 5, and more dialysate than the target replacement fluid flow rate Stf is administered.

As a factor of the pressure change of the blood that flows through the blood circuit 3 like this, the blood pressure of the patient, and the delivery amount of the blood pump 9 provided in the above-described blood circuit 3 are considered, and also, the case in which circulation of the blood is suddenly inhibited by bending of a tube composing the blood circuit 3, which is referred to as a so-called kink is considered.

When the above-described kink occurs at the upstream side of the connection portion with the above-described replacement fluid passage 5 in the above-described blood circuit 3, the pressure of the blood in the above-described connection portion becomes relatively negative pressure, and therefore, excessive dialysate flows in from the replacement fluid passage 5.

On the other hand, when the above-described kink occurs at the downstream side of the connection portion with the above-described replacement fluid passage 5, the pressure of the blood in the above-described connection portion becomes relatively positive pressure, and therefore inflow of the dialysate from the replacement fluid passage 5 is hindered.

The hemodialysis device 1 of the present embodiment quickly performs feedback control on the above-described first flow rate restriction device MV1 and the second flow rate restriction device MV2 and stabilizes the replacement fluid flow rate, even when a sudden pressure change in the blood circuit 3 like this occurs.

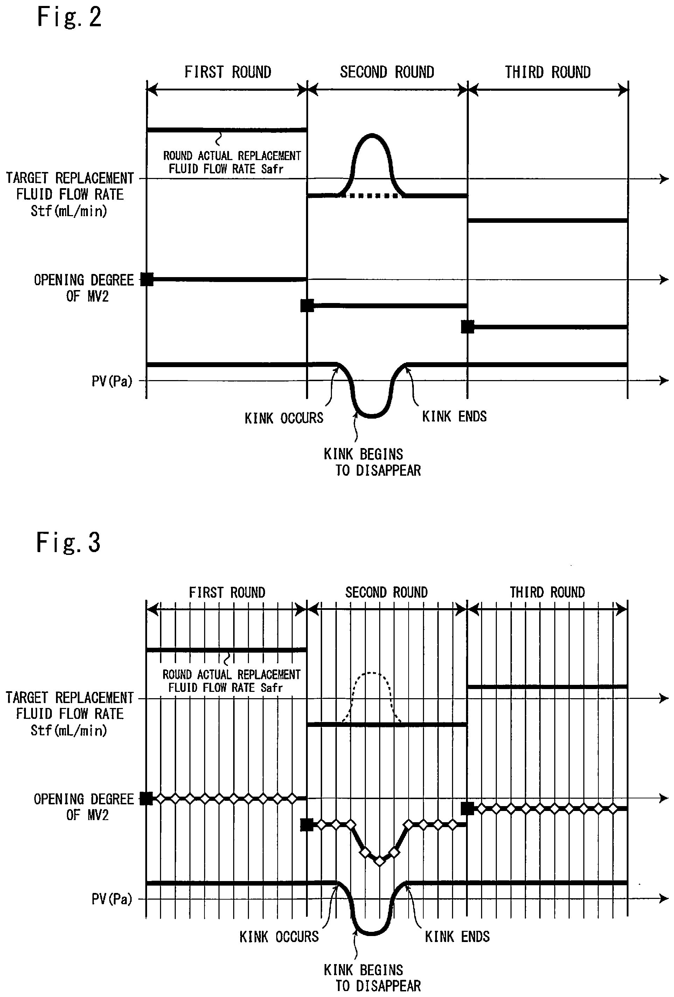

In the present embodiment, two kinds of feedback controls are performed on the above-described first and second flow rate restriction device MV1 and MV2. Figure 2 shows a case in which, of these kinds of control, only second feedback control is performed, and Figure 3 shows a case in which first feedback control and the second feedback control are performed.

In each of Figure 2 and Figure 3, a horizontal axis represents a lapse of time, and the one round from the time of the state in which the volume of the supply compartment 11A (supply compartment 12A) of the first chamber 11 (second chamber 12) becomes maximum to the time point at which the volume of the supply compartment 12A (supply compartment 11A) of the second chamber 12 (first chamber 11) becomes maximum, and supply is started is repeated from the first to a third rounds.

A vertical axis represents a flow rate (mL/min) of the replacement fluid that flows into the blood circuit 3 via the replacement fluid passage 5, an opening degree of the second flow rate restriction device MV2 provided in the replacement fluid passage 5, and a pressure (Pa) of the blood circuit 3 measured by the above-described vein-side pressure sensor PV.

The first feedback control is to perform adjustment of opening degrees of the first and second flow rate restriction device MV1 and MV2 a plurality of times during the one round and is a control that is particularly compatible with a sudden pressure change such as a kink. In Figure 3, the opening degrees of the second flow rate restriction device MV2 by the first feedback control are shown by white rhombuses.

Here, the first feedback control is executed for the purpose of stabilizing the replacement fluid flow rate in the round by performing control to maintain a round set replacement fluid flow rate Ssfr of the round that is set by the second feedback control, even when a sudden change occurs in a differential pressure of the blood circuit 3 and the dialysate circuit 4 in the round.

Here, a cause of occurrence of the sudden change to the differential pressure of the above-described blood circuit 3 and dialysate circuit 4 is a so-called kink in which the pipe composing the blood circuit 3 is bent and flow of the blood is inhibited, and also, the cause can be the patient connected to the blood circuit 3 changing the posture.

On the other hand, the second feedback control performs adjustment of the opening degrees of the first and second flow rate restriction device MV1 and MV2 for each round and updates the target dialysate flow rate Dtf and the target replacement fluid flow rate Stf for each round. In Figure 2 and Figure 3, the opening degrees of the second flow rate restriction device MV2 by the second feedback control are shown by black squares.

Here, an object of the second feedback control is as follows.

First, in order to achieve the target dialysate flow rate Dtf, and the target replacement fluid flow rate Stf that are set in advance by the doctor or the like, the opening degrees of the above-described first and second flow rate restriction device MV1 and MV2 are determined in advance.

However, when the hemodialysis device 1 is operated, the flow rates of the dialysate and the replacement fluid that actually flow deviate from the above-described target values respectively due to various factors (variations in pressure loss due to manufacturing errors of the components (electromagnetic valves, various filters, flowmeters, and the like) of the fluid circuits, manufacturing errors of the lengths of the tubes, a temperature during operation, a dialysate concentration and the like).

Thus, in the second feedback control, every time the one round ends after the dialysis treatment is started, the flow rates of the dialysate and the replacement fluid actually flow in the round are measured, and deviations between these values and the above-described target dialysate flow rate and the target replacement fluid flow rate are fed back in a next round.

Thereby, the replacement fluid flow rate is converged to the target replacement fluid flow rate Stf through each round by executing the second feedback control, although the replacement fluid flow rate deviates from the target value due to the influence of the above-described change elements at the beginning of the dialysis treatment.

First, the above-described second feedback control will be described by using Figure 2. In the control device 6, the target dialysate flow rate Dtf (mL/min) that is supplied to the dialyzer 2 via the dialysate supply passage 14, and the target replacement fluid flow rate Stf (mL/min) that is administered to the patient via the replacement fluid passage 5 are set in advance.

In the present embodiment, as an example, the target dialysate flow rate Dtf is set to 700 (mL/min), and the target replacement fluid flow rate Stf is set to 100 (mL/min).

First, the control device 6 measures the dialysate flow rate supplied toward the dialyzer 2 from the above-described dialysate supply passage 14 by the above-described flowmeter 18 and calculates a round actual dialysate flow rate Dafr (mL/min) that is an average flow rate for each round.

Furthermore, the control device 6 performs correction of a round set dialysate flow rate Dsfr based on the above-described target dialysate flow rate Dtf for each round.

Corrected round set dialysate flow rate Dsfr = target dialysate flow rate Dtf + round set dialysate flow rate Dsfr of the round - round actual dialysate flow rate Dafr       (Expression 1)

In expression 1 described above, the corrected round set dialysate flow rate Dsfr refers to the round set dialysate flow rate Dsfr set by the control device 6 in the next round, and the round set dialysate flow rate Dsfr of the round corresponds to the round set dialysate flow rate Dsfr set by the control device 6 in a previous round.

Note that when the round is a first round, the round set dialysate flow rate Dsfr of the round has a same value as the above-described target dialysate flow rate Dtf set in advance.

For example, when the round actual dialysate flow rate Dafr in the first round is 690 (mL/min) while the target dialysate flow rate Dtf is registered as 700 (mL/min), a round set dialysate flow rate Dsfr of a second round is calculated as follows by using expression 1.

700(mL/min) + 700(mL/min) - 690 (mL/min) = 710 (mL/min)

Furthermore, in the control device 6, the following calculation is performed to be used in the first feedback control described below.

Dialysate correction amount Dc = corrected round set dialysate flow rate Dsfr - target dialysate flow rate Dtf           (Expression 2)

According to expression 2, the dialysate correction amount Dc is calculated as follows.

$$710(mL/min) - 700(mL/min) = 10(mL/min)$$

When the round set dialysate flow rate Dsfr is calculated as above, the control device 6 performs feedback control on the first flow rate restriction device MV1 so as to be able to obtain the calculated round set dialysate flow rate Dsfr in the time point of start of each round.

The control device 6 calculates a dialysate pressure loss DL that is generated when the dialysate flows through the first flow rate restriction device MV1 by using expression 3 below.

Dialysate pressure loss DL = connection portion pressure PG2 - dialysate pressure PG3 - $\gamma$           (Expression 3)

Here, the connection portion pressure PG2 indicates a pressure value measured by the connection portion pressure sensor PG 2 provided in the above-described dialysate supply passage 14, and the dialysate pressure PG3 indicates a pressure value measured by the first recovery side pressure sensor PG3 provided in the above-described dialysate recovery passage 15.

Furthermore, $\gamma$ in expression 3 described above is a value obtained by adding a pressure loss (Pa) due to other elements (the flowmeter 18, the on-off valve V9, the dialyzer 2, and the like) provided in a path from the first flow rate restriction device MV1 to the connection portion pressure sensor PG2, a weight of the dialysate in the pipe composing the section, and the like.

In the second feedback control, the pressure values at the time point when one round ends are used for the above-described connection portion pressure PG2 and the dialysate pressure PG3, and therefore, the dialysate pressure loss DL calculated by using expression 3 described above is the round dialysate pressure loss DLr that is calculated for each round.

When the round dialysate pressure loss DLr is obtained as above, a dialysate pressure loss coefficient $\zeta$D is subsequently calculated by using expression 4 below.

[Expression 4]

$$\text{Dialysate pressure loss coefficient } \zeta D = \frac{\text{dialysate pressure loss} \times 2}{\text{dialysate density} \times \text{dialysate flow velocity}^2}$$

Here, a dialysate flow velocity (m/s) in expression 4 described above can be calculated by dividing the round actual dialysate flow rate Dafr measured by the above-described flowmeter 18 by a sectional area of a pipe composing the dialysate circuit 4.

Since in the second feedback control, measurement values at the time point when one round is finished are used as the above-described connection portion pressure PG2 and the dialysate pressure PG3, the dialysate pressure loss coefficient ζD calculated by using expression 4 described above is a round dialysate pressure loss coefficient ζDr that is calculated for each round.

A first command voltage V1 for controlling the above-described first flow rate restriction device MV1 can be calculated by substituting the above-described round dialysate pressure loss coefficient ζDr into expression 5 below.

First command voltage V1u = -α + ln (dialysate pressure loss coefficient ζD) + β          (Expression 5)

Here, α and β in expression 5 are constants.

Since in the second feedback control, the above-described first flow rate restriction device MV1 is controlled at the time point when one round ends, the first command voltage V1 calculated by using expression 5 described above is a round first command voltage V1r that is calculated for each round.

When the round first command voltage V1r is calculated as above, the control device 6 applies the above-described round first command voltage V1r to the above-described first flow rate restriction device MV1 at the time point of end of the round, that is, the time point of start of the next round, and controls the opening degree of the first flow rate restriction device MV1 (omitted in Figure 2).

Next, in the second feedback control, while the round first command voltage V1r for controlling the first flow rate restriction device MV1 is calculated, correction of the target replacement fluid flow rate Stf is performed based on the following procedure, and the second command voltage V2 for controlling the above-described second flow rate restriction device MV2 is calculated.

First, the control device 6 calculates a round actual replacement fluid flow rate Safr (mL/min) in one round by using expression 6 below, as the flow rate of the replacement fluid that flows through the replacement fluid passage 5.

Round actual replacement fluid flow rate Safr = dialysate total flow rate Dtf - round actual dialysate flow rate Dafr          (Expression 6)

Here, the above-described dialysate total flow rate Dtf (mL/min) can be calculated from the volume of the chamber, and the time period (fluid delivery time) required for the above-described one round. Furthermore, the round actual dialysate flow rate Dafr (mL/min) is an average flow rate in one round which is measured by the above-described flowmeter 18, which is described above.

Subsequently, the control device 6 performs correction of the round set replacement fluid flow rate Ssfr for each round by using expression 7 below.

Corrected round set replacement fluid flow rate Ssfr = Ssfr of the round + target replacement fluid flow rate Stf - round actual replacement fluid flow rate Safr          (Expression 7)

In expression 7 described above, the corrected round set replacement fluid flow rate Ssfr refers to the round set replacement fluid flow rate Ssfr that is set by the control device 6 in the next round, and the round set replacement fluid flow rate Ssfr of the round corresponds to the round set replacement fluid flow rate Ssfr set by the control device 6 in the previous round.

Note that when the round is the first round, the round set replacement fluid flow rate Ssfr of the round has a same value as the above-described target replacement fluid flow rate Stf which is set in advance.

For example, when the actual round actual replacement fluid flow rate Safr in the first round is 110 (mL/min) while the target replacement fluid flow rate Stf is registered as 100 (mL/min), the round set replacement fluid flow rate Ssfr of the second round is calculated as follows according to expression 7.

90 (mL/min) = 100 (mL/min) + 100 (mL/min) - 110 (mL/min)

That is to say, the corrected round set replacement fluid flow rate Ssfr that is set in the second round is 90 (mL/min), and the control device 6 updates the corrected round set replacement fluid flow rate Ssfr to the corrected round set replacement fluid flow rate Ssfr that is calculated (not shown), when the second round is started as shown in Figure 2.

The control device 6 also performs the following calculations for the replacement fluid flow rate to use it in the first feedback control.

Replacement fluid correction amount Sc = round set replacement fluid flow rate Ssfr - target replacement fluid flow rate Stf          (Expression 8)

According to expression 8, a replacement fluid correction amount Sc is calculated as follows.

$$-10 \ (\text{mL/min}) = 90 \ (\text{mL/min}) - 100 \ (\text{mL/min})$$

When the round set replacement fluid flow rate Ssfr is calculated as above, the control device 6 performs feedback control on the second flow rate restriction device MV2 further at the time point of start of the next round.
A replacement fluid pressure loss SL that occurs when the dialysate flows through the second flow rate restriction device MV2 is calculated by using expression 9 below.

Replacement fluid pressure loss SL = connection portion pressure PG2 - blood pressure PV + $\eta$ (Expression 9)

Here, the connection portion pressure PG2 indicates the pressure value measured by the connection portion pressure sensor PG2 provided in the above-described dialysate supply passage 14, and the blood pressure PV indicates the pressure value measured by the vein-side pressure sensor PV provided in the vein-side passage 8 of the above-described blood circuit 3.
Furthermore, $\eta$ in expression 9 described above is a value obtained by adding a pressure loss of the other elements (the on-off valve V10, the connection port 19 and the like) provided in a path from the second flow rate restriction device MV2 to the vein-side pressure sensor PV, a weight of the dialysate in a pipe composing the section, and the like.
Furthermore, in the second feedback control, since the pressure values at the time point when one round ends are used for the above-described connection portion pressure PG2 and the blood pressure PV, the replacement fluid pressure loss SL calculated by using expression 9 described above is a round replacement fluid pressure loss SLr which is calculated for each round.
When the round replacement fluid pressure loss SLr is obtained as above, a replacement fluid pressure loss coefficient $\zeta$S is subsequently calculated by using expression 10 below.

[Expression 10]

$$\text{Replacement fluid pressure loss coefficient } \zeta S = \frac{\text{replacement fluid pressure loss} \times 2}{\text{dialysate density} \times \text{replacement fluid flow velocity}^2}$$

Here, a replacement fluid flow velocity can be calculated by dividing the above-described calculated round actual replacement fluid flow rate Safr by a sectional area of a pipe composing the dialysate circuit 4.
In the second feedback control, since the pressure values at the time point when one round ends are used as the above-described connection portion pressure PG2 and the blood pressure PV, the replacement fluid pressure loss coefficient $\zeta$S calculated by using expression 10 described above is a round replacement fluid pressure loss coefficient $\zeta$Sr that is calculated for each round.
The second command voltage V2 for controlling the above-described second flow rate restriction device MV2 can be calculated by substituting the above-described round replacement fluid pressure loss coefficient $\zeta$Sr into expression 11 below.

Second command voltage V1 = -$\delta$ + in (replacement fluid pressure loss coefficient $\zeta$S) + $\varepsilon$ (Expression 11)

Here, $\delta$ and $\varepsilon$ in expression 11 are constants.
In the second feedback control, the above-described second flow rate restriction device MV2 is controlled at the time point when one round ends, and the second command voltage V2 calculated by using expression 11 described above is a round second command voltage V2r that is calculated for each round.
When the round second command voltage V2r is calculated as above, the control device 6 applies the above-described round second command voltage V2r to the above-described second flow rate restriction device MV2 at the time point of start of the next round, as in the control of the above-described first flow rate restriction device MV1, and the opening degree of the second flow rate restriction device MV2 is controlled (black squares in Figure 2).
As described above, the above-described second feedback control updates the round set dialysate flow rate Dsfr and the round set replacement fluid flow rate Ssfr based on the dialysate flow rate and the replacement fluid flow rate in the relatively long time of one round, and with this, updates the opening degrees of the first and the second flow rate restriction device MV1 and MV2.

Here, an operation when only the second feedback control is performed will be described by using Figure 2.

First, in the first round, for the reason as described above, that is, due to the variation in pressure loss and the like by the components or the like, the round actual replacement fluid flow rate Safr in reality becomes large with respect to the target replacement fluid flow rate Stf.

Thus, the control device 6 performs the aforementioned second feedback control, sets the corrected round set replacement fluid flow rate Ssfr in the second round, and sets the opening degree of the second flow rate restriction device MV2.

In Figure 2, the opening degree of the second flow rate restriction device MV2 is controlled to be restricted to a narrower opening degree than the opening degree in the first round, and thereby control is performed so that the actual replacement fluid flow rate Saf in the second round becomes less than the actual replacement fluid flow rate Saf in the first round.

By repeating the above-described second feedback control for each round, it is possible to converge the round actual replacement fluid flow rate Safr in which a deviation occurs at the time point of start of dialysis treatment to the target replacement fluid flow rate Stf.

However, with only the second feedback control, there arises a problem that quick convergence to the target replacement fluid Stf cannot be achieved when a sudden pressure change such as a kink occurs in the blood circuit 3.

Here, as an example, a case in which a kink occurs at the upstream side of the connection portion of the blood circuit 3 and the replacement fluid passage 5 in the second round will be described.

When a kink occurs at the upstream side of the connection portion, a downstream side of the connection portion in the blood circuit 3 becomes relatively negative pressure, and a pressure difference between the dialysate circuit 4 and the blood circuit 3 increases, so that much replacement fluid flows in from the replacement fluid passage 5.

Since in the second feedback control, the opening degree of the second flow rate restriction device MV2 that is set at the time point of start of the second round is maintained, a large amount of dialysate flows into the blood circuit 3 from the replacement fluid passage 5.

Thereafter, when the kink is eliminated, the actual replacement fluid amount Saf decreases, but the round actual replacement fluid flow rate Safr in the second round becomes larger than the target replacement fluid flow rate Stf.

Then, the control device 6 sets the corrected round set replacement fluid flow rate Ssfr in the third round, but the value is less than the target replacement fluid flow rate Stf, so that the opening degree of the second flow rate restriction device MV2 in the third round is restricted more than the opening degree at the time of the second round.

As a result, the round actual replacement fluid flow rate Safr in the third round is a value that is lower than and deviates from the target replacement fluid flow rate Stf, and in this case, the control device 6 sets the opening degree of the second flow rate restriction device MV2 in a fourth round to be large.

Then, the round actual replacement fluid flow rate Safr in the fourth round is larger than the target replacement fluid flow rate Stf, so that in this manner, even if the control device 6 intends to converge the round actual replacement fluid flow rate Safr to the target replacement fluid flow rate Stf by the second feedback control, it takes a long time until the round set replacement fluid flow rate Ssfr converges to the target replacement fluid flow rate Stf, and the replacement fluid flow rate does not stabilize.

On the other hand, when a sudden pressure change in the blood circuit 3 such as a kink occurs, the first feedback control quickly stabilizes the replacement fluid flow rate by updating the opening degrees of the first and second flow rate restriction device MV1 and MV2 a plurality of times during one round.

In Figure 3, in the first feedback control, the control device 6 updates the opening degree of the first flow rate restriction device MV1 every 2 seconds. The opening degree at this time is calculated based on the corrected round set dialysate flow rate Dsfr (the target dialysate flow rate Dtf in the case of the first round) that is calculated in the above-described second feedback control in the round.

Note that an update interval is not limited to 2 seconds and can be arbitrarily set if the update can be performed a plurality of times in one round.

As described in detail below, the control device 6 performs feedback control as follows regarding the opening degree of the first flow rate restriction device MV1 after the two seconds.

First, a dialysate pressure loss DL generated when the dialysate flows through the first flow rate restriction device MV1 is calculated by using expression 12 described below.

$$\text{Dialysate pressure loss DL} = \text{connection portion pressure PG2} - \text{dialysate pressure PG3} - \gamma \qquad \text{(Expression 12)}$$

Note that the connection portion pressure PG2, and the dialysate pressure PG3 are pressure values that are measured for every unit time, and the dialysate pressure loss DL obtained as above is a unit time dialysate pressure loss DLu.

When the unit time dialysate pressure loss DLu is obtained as above, a dialysate pressure loss coefficient $\zeta$D is subsequently calculated by using expression 13 described below.

[Expression 13]

$$\text{Dialysate pressure loss coefficient } \zeta \text{D} = \frac{\text{dialysate pressure loss} \times 2}{\text{dialysate density} \times \text{dialysate flow velocity}^2}$$

Here, the dialysate flow velocity in expression 13 described above is calculated by using the corrected round set dialysate flow rate Dsfr calculated in the above-described second feedback control in the round. This is because in the present embodiment, the dialysate flow rate is calculated for each round by using the above-described flowmeter 18, and therefore the flow rate for each unit time cannot be measured.

Here, the dialysate pressure loss coefficient $\zeta$D is calculated by the connection portion pressure PG2 and the dialysate pressure PG3 for each unit time, and the corrected round set dialysate flow rate Dsfr in the round. The dialysate pressure loss coefficient $\zeta$D obtained as above is a unit time dialysate pressure loss coefficient $\zeta$Du.

The first command voltage V1 for controlling the above-described first flow rate restriction device MV1 can be calculated by substituting the above-described unit time dialysate pressure loss coefficient $\zeta$Du into expression 14 described below.

First command voltage V1u = -$\alpha$ + ln (dialysate pressure loss coefficient $\zeta$D) + $\beta$    (Expression 14)

As described above, since the unit time dialysate pressure loss coefficient $\zeta$Du is calculated for each unit time, the first command voltage V1 calculated by using expression 14 described above is a unit time first command voltage V1u calculated for each unit time.

As above, the control device 6 calculates the unit time first command voltage V1u every unit time, and applies the above-described unit time first command voltage V1u to the above-described first flow rate restriction device MV1, and thereby the opening degree of the first flow rate restriction device MV1 is controlled every unit time (omitted in Figure 2).

Similarly to the second feedback control, in the first feedback control, a unit time second command voltage V2u for controlling the above-described second flow rate restriction device MV2 is calculated to perform correction of the replacement fluid flow rate.

In the present embodiment, the control device 6 updates the opening degree of the above-described second flow rate restriction device MV2 every 1 second, and the opening degree is calculated based on the corrected round set replacement fluid flow rate Ssfr (the target replacement fluid flow rate Stf in the case of the first round) that is calculated in the above-described second feedback control in the round.

Note that an update interval is not limited to 1 second, and can be arbitrarily set if update can be performed a plurality of times in one round.

As described in detail below, regarding the opening degree of the second flow rate restriction device MV2 after 1 second, the control device 6 performs feedback control as follows.

First, the replacement fluid pressure loss SL that is generated when the dialysate flows through the second flow rate restriction device MV2 is calculated by using expression 15 described below.

Replacement fluid pressure loss SL = connection portion pressure PG2 - blood pressure PV - $\eta$    (Expression 15)

Here, the connection portion pressure PG2 and the blood pressure PV are pressure values per second, and the replacement fluid pressure loss SL calculated by using expression 9 described above is a unit time replacement fluid pressure loss SLu that is calculated every unit time.

When the unit time replacement fluid pressure loss SLu is obtained as above, the replacement fluid pressure loss coefficient $\zeta$S is subsequently calculated by using expression 16 described below.

[Expression 16]

$$\text{Replacement fluid pressure loss coefficient } \zeta\, S = \frac{\text{replacement fluid pressure loss} \times 2}{\text{dialysate density} \times \text{replacement fluid flow velocity}^2}$$

Here, the replacement fluid flow velocity in expression 16 described above is calculated by using the corrected round set replacement fluid flow rate Ssfr that is calculated in the above-described second feedback control in the round. This is because the actual replacement fluid flow rate is calculated for each round, and therefore the flow rate per unit time cannot be measured, as described above.

Here, since the dialysate pressure loss coefficient ζD is calculated by the connection portion pressure PG2, the blood pressure PV, and the unit time set replacement fluid flow rate Ssfu per unit time, the replacement fluid pressure loss coefficient ζS obtained as above is a unit time replacement fluid pressure loss coefficient ζSu.

The second command voltage V2 for controlling the above-described second flow rate restriction device MV2 can be calculated by substituting the above-described unit time replacement fluid pressure loss coefficient ζSu into expression 17 described below.

Second command voltage V1 = -δ + in (replacement fluid pressure loss coefficient ζS) + ε        (Expression 17)

In the first feedback control, the second flow rate restriction device MV2 is controlled every 1 second, and the second command voltage V2 calculated by using expression 17 described above is a unit time second command voltage V2u that is calculated every unit time.

When the unit time second command voltage V2u is calculated as above, the control device 6 applies the above-described unit time second command voltage V2u to the above-described second flow rate restriction device MV2, and controls the opening degree of the second flow rate restriction device MV2 every unit time (white rhombuses in Figure 3).

A case in which the first feedback control and the second feedback control are performed, which is shown in Figure 3, will be described. Here, as in the case of Figure 2, feedback control in the case in which a kink occurs at the upstream side of the connection portion with the replacement fluid passage 5 in the blood circuit 3 midway through the second round will be described.

As in Figure 2, though the pressure of the connection portion of the blood circuit 3 and the replacement fluid passage 5 suddenly lowers, and the actual replacement fluid flow rate temporarily rises directly after occurrence of the kink, the rise of the actual replacement fluid flow rate is quickly restrained by the control device 6 performing the above-described first feedback control that changes the opening degree of the second flow rate restriction device MV2 every unit time.

As a result, it is possible to bring the actual replacement fluid flow rate in the second round to the round set replacement fluid flow rate Ssfr of the second round, which is corrected by the above-described second feedback control.

Accordingly, the round set replacement fluid flow rate Ssfr in the third round is converged to the target replacement fluid flow rate Stf as compared with the case in which only the second feedback control is performed shown in Figure 2.

That is to say, by performing the first feedback control together with the second feedback control, it is possible to quickly stabilize the replacement fluid flow rate even when a sudden change occurs to the differential pressure between the blood circuit 3 and the dialysate circuit 4.

Note that although in the above-described embodiment, the first feedback control and the second feedback control are performed, the second feedback control may be omitted, and the feedback may be performed with only the first feedback control.

In this case, the control device 6 can control the first and second flow rate restriction device MV1 and MV2 so as to converge to the target replacement fluid flow rate Stf that is set in advance, without setting the round set replacement fluid flow rate Ssfr for each round.

Furthermore, in the above-described embodiment, the above-described control device performs the first feedback control or the second feedback control by using the aforementioned expressions, but other than this, for each replacement fluid flow rate set as the target (the target replacement fluid flow rate Stf or the round set replacement fluid flow rate Ssfr), relationship of the differential pressure of the connection portion pressure PG2 and the blood pressure PV, and the opening degrees (command voltages) of the first and second flow rate restriction device MV1 and MV2 at that time may be measured, and may be stored in a required table.

In this case, when the connection portion pressure PG2 and the blood pressure PV are measured during dialysis treatment, the control device 6 can read the command voltage corresponding to the target dialysate flow rate Dtf or the round set dialysate flow rate Dsfr at this time from the above-described table, and can control the opening degrees of the first flow rate restriction device MV1 and the second flow rate restriction device MV2.

[Reference Signs List]

| 1 | hemodialysis device | 2 | dialyzer |
|---|---|---|---|
| 3 | blood circuit | 4 | dialysate circuit |
| 5 | replacement fluid passage | 6 | control device |
| 7 | artery-side passage | 8 | vein-side passage |
| 14 | dialysate supply passage | 15 | dialysate recovery passage |

| | |
|---|---|
| MV1 | first flow rate restriction device MV2 second flow rate restriction device |
| PV | vein-side pressure sensor (blood circuit pressure measurement device) |
| PG2 | connection portion pressure sensor (connection portion pressure measurement device) |
| Dtf | target dialysate flow rate |
| Stf | target replacement fluid flow rate |
| Safr | round actual replacement fluid flow rate |
| Ssfr | round set replacement fluid flow rate |
| Sc | replacement fluid correction amound |
| SLr | round replacement fluid pressure loss |
| $\zeta$Sr | round replacement fluid pressure loss coefficient |
| Ssfu | unit time set replacement fluid flow rate |
| SLu | unit time replacement fluid loss |
| $\zeta$Su | unit time replacement fluid pressure loss coefficient |

**Claims**

1. A hemodialysis device comprising a dialyzer that performs hemodialysis, a chamber that is provided in a dialysate circuit and stores a dialysate, a dialysate supply passage that is connected to a supply compartment of the chamber to supply a fresh dialysate to the dialyzer, a dialysate recovery passage that is connected to a recovery compartment of the chamber to recover a used dialysate that flows through the dialyzer, a blood circuit that causes blood to flow through the dialyzer, a replacement fluid passage provided between the dialysate supply passage and the blood circuit, first flow rate restriction device provided at a downstream side of a connection portion with the replacement fluid passage in the dialysate supply passage, second flow rate restriction device provided in the replacement fluid passage, and control device that controls the first flow rate restriction device and the second flow rate restriction device,

   the control device adjusting a flow rate of a replacement fluid that is supplied to the blood circuit via the replacement fluid passage by controlling the first flow rate restriction device and the second flow rate restriction device, **characterized by** comprising
   connection portion pressure measurement device that measures a pressure of the connection portion of the dialysate supply passage and the replacement fluid passage, and blood circuit pressure measurement device that measures a pressure of the blood circuit, and **characterized in that**
   during one round from a time when the supply compartment of the chamber is in a state of being filled with the fresh dialysate until all of the fresh dialysate in the supply compartment is discharged, the control device
   performs feedback control that adjusts the second flow rate restriction device a plurality of times based on a replacement fluid flow rate set as a target, a measured pressure of the connection portion pressure measurement device, and a measured pressure of the blood circuit pressure measurement device.

2. The hemodialysis device according to claim 1, **characterized in that**

in addition to first feedback control executed a plurality of times during the one round, the control device, sets a round set replacement fluid flow rate of a next round that is set as a target in the next round based on a round actual replacement fluid flow rate in the round, and a round set replacement fluid flow rate of the round that is set as a target in the round, and

performs second feedback control that adjusts the first and second flow rate restriction device based on a measured pressure of the connection portion pressure measurement device and a measured pressure of the blood circuit pressure measurement device at a time of an end of the round.

EP 4 609 889 A1

Fig. 1

15

Fig. 2

Fig. 3

## INTERNATIONAL SEARCH REPORT

| International application No. |
| --- |
| **PCT/JP2022/039439** |

| A. | CLASSIFICATION OF SUBJECT MATTER |
| --- | --- |

*A61M 1/16*(2006.01)i
FI:  A61M1/16 110

According to International Patent Classification (IPC) or to both national classification and IPC

| B. | FIELDS SEARCHED |
| --- | --- |

Minimum documentation searched (classification system followed by classification symbols)

A61M1/16

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2001-112863 A (KITA KYUSHU BIOPHYSICS KENKYUSHO KK) 24 April 2001 (2001-04-24) entire text, all drawings | 1-2 |
| A | JP 2021-62067 A (SHIBUYA KOGYO CO LTD) 22 April 2021 (2021-04-22) entire text, all drawings | 1-2 |
| A | JP 2020-81338 A (SHIBUYA KOGYO CO LTD) 04 June 2020 (2020-06-04) entire text, all drawings | 1-2 |
| A | JP 2007-510473 A (BAXTER INTERNATIONAL INC.) 26 April 2007 (2007-04-26) entire text, all drawings | 1-2 |
| A | WO 2021/044545 A1 (JAPAN LIFELINE CO LTD) 11 March 2021 (2021-03-11) entire text, all drawings | 1-2 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **22 November 2022** | **06 December 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2022/039439**

| Patent document cited in search report | | | Publication date (day/month/year) | Patent family member(s) | | | Publication date (day/month/year) |
|---|---|---|---|---|---|---|---|
| JP | 2001-112863 | A | 24 April 2001 | (Family: none) | | | |
| JP | 2021-62067 | A | 22 April 2021 | (Family: none) | | | |
| JP | 2020-81338 | A | 04 June 2020 | EP | 3656413 | A1 | |
| | | | | CN | 111202879 | A | |
| JP | 2007-510473 | A | 26 April 2007 | US | 2005/0131332 | A1 | |
| WO | 2021/044545 | A1 | 11 March 2021 | CN | 114340692 | A | |

Form PCT/ISA/210 (patent family annex) (January 2015)

**EP 4 609 889 A1**

**Patent documents cited in the description**

- JP 2021062067 A